# EUROPEAN PATENT SPECIFICATION

(11) **EP 0 611 561 B1**
(45) Date of publication and mention of the grant of the patent: **02.12.1998**
(21) Application number: 93307597.0
(22) Date of filing: 24.09.1993
(51) Int. Cl.: A61F 5/00, A61B 17/12, A61F 2/00

(54) **Laparoscopic adjustable gastric banding device**
Laparoskopisches einstellbares Magenband
Bande gastrique ajustable pour laparoscopie

(30) Priority: 18.02.1993 US 19302; 07.04.1993 US 43872; 12.05.1993 US 59592
(43) Date of publication of application: 24.08.1994
(73) Proprietor: Kuzmak, Lubomyr Ihor, South Orange, New Jersey 07079 (US)
(72) Inventor: Kuzmak, Lubomyr Ihor, South Orange, New Jersey 07079 (US)
(74) Representative: Linn, Samuel Jonathan

(56) References cited:
- WO-A-86/04498
- DE-C- 4 133 800
- US-A- 5 074 868

## Description

The present invention relates to a device for the treatment of morbid obesity, and, in particular, relates to gastric banding devices which encircle a portion of the stomach to form a stoma opening of reduced diameter so as to restrict food intake.

In the previously known stoma adjustable silicone gastric banding (SASGB), disclosed in U.S. Patent No. 4,592,339 (Kuzmak et al.), an inflatable or expandable section of a gastric band covers a portion of the circle defined by the band. The expandable section of the device permits some adjustment of the size of the stoma opening both intraoperatively and postoperatively. This device is simple in construction and enables the desired diameter of the stoma opening to be maintained. A similar gastric banding device is disclosed in WO 86/04498. U.S. Patent No. 4,696,288 (Kuzmak et al.) discloses a calibrating apparatus which facilitates controlling the size of the stoma with a gastric band.

Even with the ability to adjust the stoma size, it may still be desirable in given situations, e.g., obstruction of the stoma from edema and migration of the band to remove the band entirely when deflation is insufficient to relieve the condition. A scheme for enabling gastric bands to be removed is discussed in U.S. Patent No. 5,074,868 (Kuzmak). When a remotely situated pull cord is retracted, a suture cutting blade severs sutures securing overlapping portions of the band which encircle the stomach. Another removable gastric band device is disclosed in U.S. Patent No. 5,160,338 (Vincent).

A laparoscopic gastric band and a method for using such a band is disclosed in U.S. Serial No. 07/719,123, filed June 20, 1991, in the name of Lubomyr I. Kuzmak. Reference is also made to Lubomyr I. Kuzmak, "*Stoma Adjustable Silicone Gastric Banding*," Problems in General Surgery, Volume 9, No. 2, J.B. Lippincott Co., April/June 1992.

Although the devices disclosed in the Kuzmak and Kuzmak at al patents and patent application referred to above have proven to be successful in practice, some potential disadvantages remain. For example, the belt-like band construction of the devices wherein one end of the band fits through a buckle at the other end can be difficult to tighten, and bulges are created at the ends of the inflated or expanded section (caused by the fact that the expanded section only partially extends around the circumference of the band), thereby resulting in localized compression of the stomach wall. Further, the parts of the band must be sutured together when the band is placed at a desired position around the stomach and this suturing can be difficult to carry out.

The present invention seeks to provide a laparoscopic SASGB which is more effective in providing uniform modification of the stoma size, is less invasive to the stomach wall, and is also easily reversible.

The present invention also seeks to provide a laparoscopic gastric band positioned around the stomach which includes a locking element secured by a remotely removable device which eliminates or substantially reduces the discomfort caused to some patients by related devices.

The present invention aims to eliminate bulging associated with SASGB devices of the prior art and thus eliminate localized compression of the stomach wall.

The present invention also aims to eliminate the need for suturing of the band together in order to secure the band at a desired position around the stomach.

In addition the present invention seeks to provide a SASGB having an increased range of stoma adjustment.

In accordance with the invention, a gastric banding device is provided for forming a stoma opening in a stomach so as to restrict food intake to the lower digestive portion of the stomach and which is particularly adapted for use in, but not limited to, laparoscopic implantation and removal of the device. The gastric banding device according to the invention is as defined in claim 1 hereof. According to a preferred embodiment thereof, the device comprises: an elongate band member including a band portion for, in use, encircling a stomach to form a stoma opening in the stomach, the band member including a free end; an expandable section secured to the band portion of the band member for varying the size of the stoma opening; a securing member located near the free end of the band member and protruding outwardly therefrom; a recess, formed in an intermediate portion of the band member segment having a thickness greater than the thickness of the remainder of the band member, for receiving the securing member therein; and means for retaining the securing member in the recess so that the band portion forms a circle of a predetermined fixed diameter around the stoma opening.

Advantageously, the retaining means comprises a moveable threaded bolt, and in a preferred embodiment, the securing member includes a threaded hole therein in which the bolt is received. The bolt is preferably formed by a distal part of an elongate control element located within the band member and extending longitudinally thereof. The control element includes a proximal end which extends beyond the band member for allowing remotely controlled release of the securing member from the recess by movement of the bolt. Advantageously, the device further includes a guiding plate, located within the band member on one side of the recess, for guiding the movement of the bolt, and a holding plate located within the band member on the other side of the recess, for retaining the free end of the bolt.

The expandable section of the device of the invention comprises an expandable member corresponding to that discussed above, i.e., one extending around substantially the entire circumference of the band portion, i.e., the portion that, in use, encircles the stomach wall.

A remotely disposed fluid injection unit is preferably provided which is in fluid connection with the expandable section through the band member such that the size of the stomach opening can be altered by adding or withdrawing fluid to cause expansion and retraction of the expandable section.

Advantageously, a barrier means is provided for preventing tissue from growing around the securing member. The barrier means preferably comprises a soft sponge-like silicone barrier element.

Preferably, a thin loop is formed around the securing member for ensuring a secure fit between the securing member and the recess.

In accordance with a further aspect of the present disclosure, a method is provided for removing a band a method is provided for removing a band having an expandable section encircling a stomach which forms a circle of a predetermined fixed diameter by employing a securing means to retain a locking element projecting from said band at a location near the distal end of said band into a recess formed at an intermediate location along said band, the method comprising the steps of: retracting the securing means so as to release the locking element from the recess; cutting the band close to the recess on a side of the recess opposite the expandable section, the securing means being sufficiently retracted such that it is not severed by the cutting; removing the cut portion not containing the recess and the expandable section through a small incision; and laparoscopically removing the remainder of the band containing the recess and the expandable section.

In accordance with an additional aspect of the present disclosure, a method is provided for removing a band encircling a stomach which forms a circle of a predetermined fixed diameter by employing a securing means to retain a locking element projecting form said band at a location near the distal end of said band into a recess formed at an intermediate location along said band, the method comprising the steps of: completely removing the securing means through a small incision, thus also releasing the locking element from the recess; and laparoscopically removing the band.

Preferably, for either method described above, the further step of removing an injection port in communication with the expandable section through the small incision prior to the laparoscopically removing step is also performed.

In accordance with yet another aspect of the present disclosure a method is provided for laparoscopically implanting a gastric band device including an encircling band portion which, in use, encircles a stomach so as to form a restricted stoma opening for limiting food intake to the lower digestive portion of the stomach, the gastric band device including an expandable section secured to the encircling band portion for varying the size of the stomach opening, an elongate band portion incorporating a remotely operable securing means for, when the device is implanted, retaining a locking element projecting from the gastric band device at a location near the distal end of the encircling band portion in a locking position wherein the locking element is received in a recess formed at an intermediate location along the gastric banding device so that, when the band is implanted, the encircling band portion forms a circle of a predetermined fixed diameter around the stoma opening, and expansion control means, communicating with the expandable section through a channel formed in the elongate band portion, for controlling expansion and contraction of said expandable section, said method comprising the steps of:
using an endoscope introduced into an cannula to dissect tissue surrounding the stomach to provide a path for the elongate band portion of the gastric band device;
laparoscopically introducing the gastric band into the abdominal cavity such that said encircling band portion encircles the stomach and said locking element is inserted in said recess so that said encircling band portion forms a circle of predetermined fixed diameter around said stoma opening of the stomach;
operating said securing means to lock said locking element in said recess; and
implanting said expansion control means in the abdominal muscle wall.

Other features and advantages of the invention will be set forth in, or apparent from, the following detailed description of preferred embodiments of the invention.

The invention will now be described in further detail with reference to the accompanying drawings, wherein:
Figure 1 is a perspective view of the modified laparoscopic SASGB device of the present invention as placed in position around a stomach;
Figure 2(a) is a side view of the SASGB device constructed in accordance with a preferred embodiment of the present invention, showing the device in an extended, rest state.
Figure 2(b) is a cross sectional view of a detail of Figure 2(a) showing the locking recess or slot;
Figure 3(a) is a top plan view of the device of Figure 2(a);
Figure 3(b) is a side view of the locking element of the device of Figure 2(a);
Figure 4 is a side view of the device of Figure 2(a) with the band locked in place to form a circle or loop;
Figure 5(a) is a side view of the device shown in Figure 2(a) with the band unlocked; and
Figure 5(b) is a side view of the device shown in Figure 2(a) with the band unlocked and the locking bolt and associated control member completely removed.

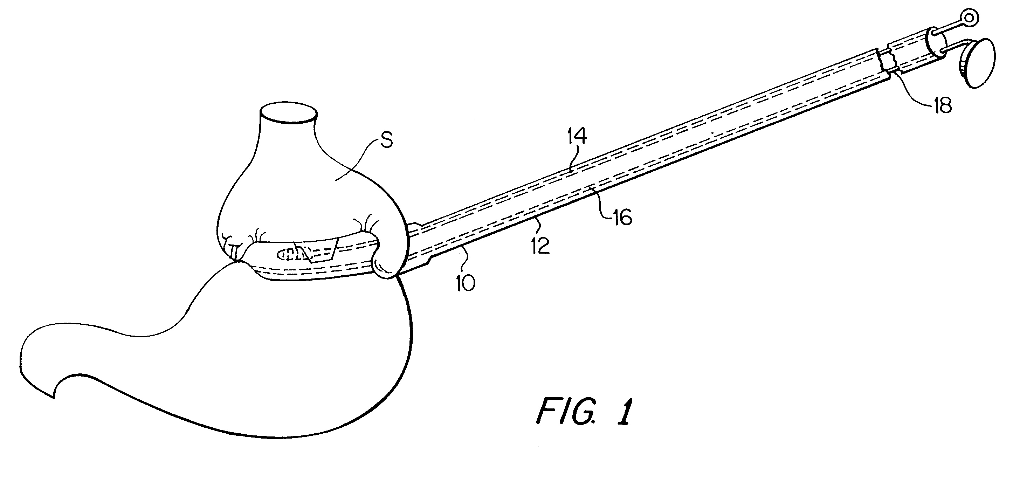

Referring to Figure 1, a SASGB device, generally denoted 10, is shown in place around a stomach S. More particularly, the device 10 comprises a band 12 which includes a loop or encircling portion (which is not fully visible in Figure 1) that, in use, fully encircles the stomach S so as to form stoma opening at the loop portion, thereby restricting food intake to the lower digestive portion of the stomach S. As described in more detail below, a channel 14 allows fluid to be transmitted to or removed from an expandable section or portion of the device 10 which is not visible in Figure 1, while a further channel 16 contains an elongate control member 18 for a securing or locking mechanism for encircling loop.

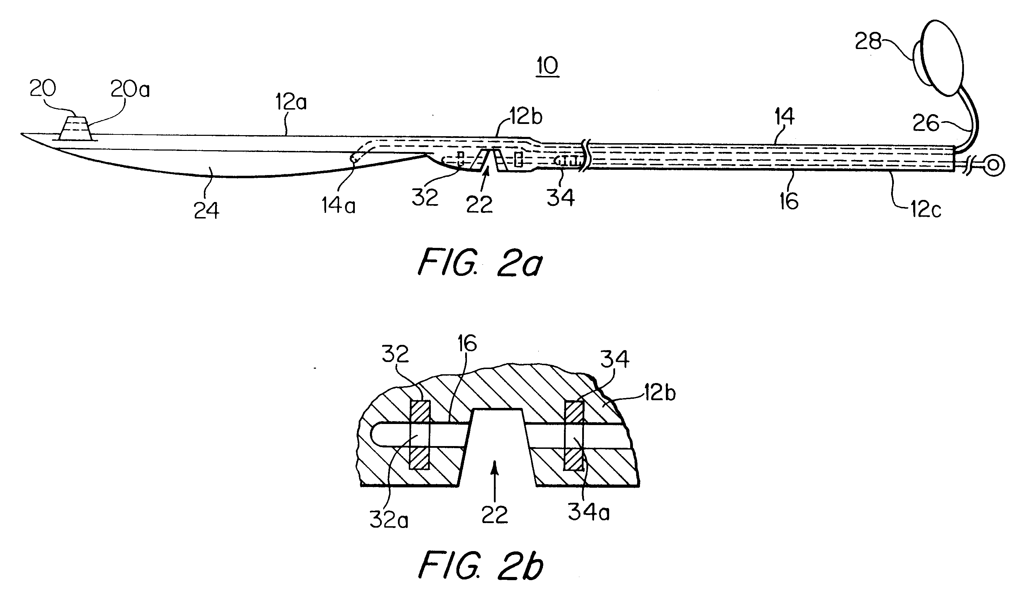

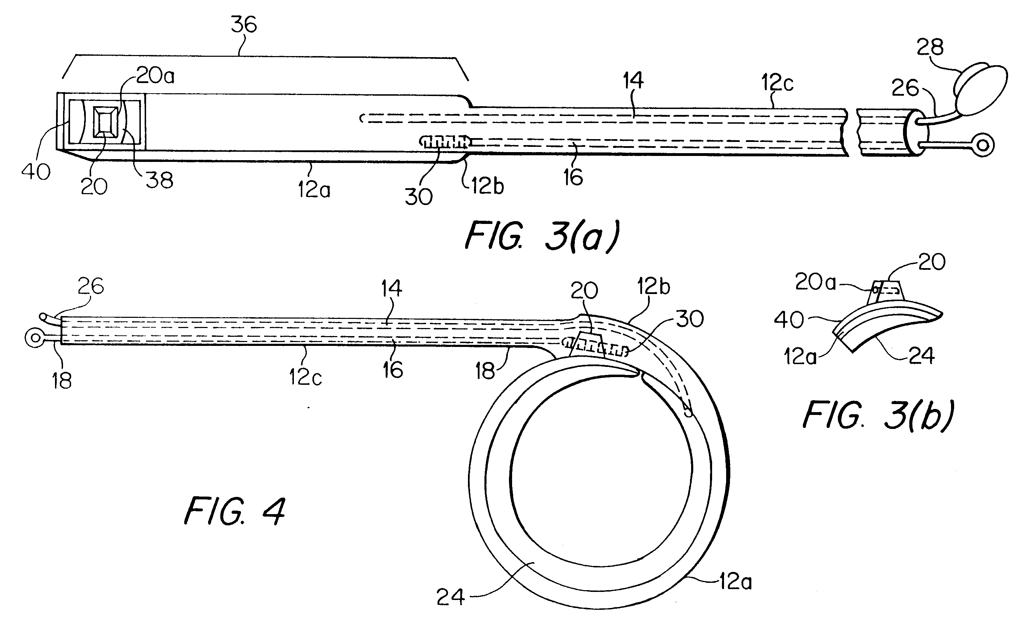

The SASGB device 10 of Figure 1 is shown in more detail in Figures 2 to 5. As shown in these figures, the band locking device referred to above includes an outwardly projecting securing or locking member 20 located towards the tip or free (distal) end of the band 12 and a recess or slot 22 in which locking member 20 is received, as described hereinafter. The abovementioned expandable or inflatable section of the band 12 is denoted 24, and extends along the entire band portion 12a which, in use, encircles the stomach, as is perhaps best seen in Figure 4. The channel 14 itself forms a passage, or accommodates a separate tube 26, which connects the expandable or inflatable section 24 of the band 12 with an injection unit 28 for injecting or removing solution in order to adjust the size of the stoma opening. This solution is a physiologically compatible fluid, such as saline.

Considering the securing or locking mechanism in more detail, the recess or slot 22 located in an intermediate band portion 12b is shaped to accommodate locking member or element 20 therein when the distal end of band 12 is formed into a loop as shown in Figure 4. The locking element 20 has a hole 20a therein through which a screw threaded locking bolt 30 formed at the end of the control member 18 introduced through channel 16 (and best seen in Figure 4) is inserted, thus securing the locking member 20 in the recess or slot 22. As can best be seen in Figure 2(b), a first, holding or retaining plate 32 having an opening 32a therein is provided near the free or distal end of channel 16 adjacent to recess 22 to hold the tip of the threaded screw bolt 30. Plate 32 is bonded into the band 12. The opening 32a in the plate 32 is sized to fit or match the shape of the tip of the screw bolt 30 and is not threaded. A second, guiding plate 34 has an opening 34a therein the diameter of which equals the diameter of the bolt 30 without the screw thread. Plate 34 is bonded into band 12 on the other side of the slot 22. The control member 18, which terminates at the distal end thereof in the threaded locking bolt 30, extends at the opposite proximal end thereof beyond the length of the band 12, thus enabling movement of bolt 30, and hence locking of the distal end of the band 12 in recess 22, to be controlled remotely.

As is shown in Figures 2(a) and 3(a), the band thickness of the receiving segment or portion of the band 12b is greater than the thickness of the remainder of the band. This increased thickness is necessary in order to accommodate the thickness of the locking element or projection 20 formed near the distal end of the band, while still allowing channel 14 to reach the inflatable portion 24.

In a specific, non-limiting but advantageous embodiment, the band locking element 20 and the band 12 itself are preferably made of silicone and, as described above, the former has an opening 20a with a screw type thread for locking the band 12 in place around the stomach. The proximal edge of the band locking element 20 is preferably 1 1/2 centimeters from the tip or free end of the band. Further, the band locking element 20 is preferably of the truncated polygonal shape shown in the drawings and has a height of 4 millimeters and a length at its base of 6 millimeters. The inflatable section or part 24 is preferably reinforced and in the exemplary embodiment under consideration, is 8 centimeters in length. Reinforcement of the sides of the inflatable part 24 is important in order to eliminate side bulging and to increase the circular inflation which is important in providing stoma calibration. The silicone covering the band 12 may be used for reinforcement. The tube 26 located within channel 14 is also preferably made of silicone. The retaining plate 32 and the guiding plate 34 are preferably made of silicone and are located 1 1/2 centimeters from the recess or slot 22 on opposite sides thereof. Bolt 30 is advantageously made of silicone or plastic as is control element 18.

The band 12 is, as stated, preferably made of silicone and is wider than previously used bands, advantageously 13mm. The band thickness of the portion of the band 12a covered by the inflatable section 24, i.e., that which encircles the stomach, is similar in thickness to previous bands, preferably 2mm. The band thickness of the receiving segment is preferably between 4 to 6 mm. The band thickness of the elongate band portion 12c is preferably between 2 to 4 mm, and is roughly one-half to two-thirds of the thickness of the receiving segment. The front part of the band 12, i.e., band portions 12a and 12b, indicated by bracket 36, is preferably reinforced with dacron mesh. The remaining elongate band portion 12c is preferably not reinforced so that it is soft and flexible.

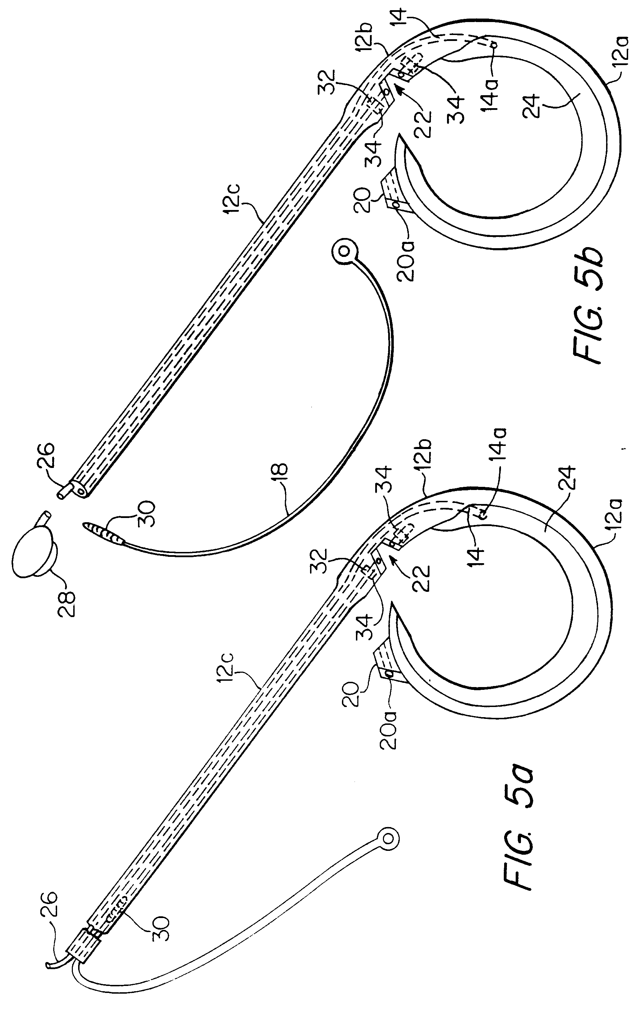

In order to better accommodate the positioning of the band parts in the locking position, a thin loop of silicone, indicated at 38 in Fig. 3(a), is bonded around the projecting barrier or locking element 20. Additionally, a soft sponge-like silicone guard portion, indicated at 40 in Figure 3(a) and Figure 3(b), is advantageously placed around the projecting locking element 20 in order to prevent the capsule tissue, i.e., tissue forming a capsule around the band, from growing into that space. Such capsule tissue may cause some limited resistance when the band is removed without major surgery. Linear markings (not shown) may be placed on both parts of the band 12 that are joined together as shown in Figures 4, 5(a) and 5(b) so that when the markings are matched with each other, an indication is provided that the locking parts are in correct position. As noted above, Figure 4 shows the device in the fully locked position with the bolt 30 fully extended through the projection locking element 20 and into the holding or retaining plate 32. The diameter of the "locked" band, i.e., the band in the closed position shown in Figure 4, is preferably 4 cm, although a diameter between about 4 to 6 cm could be acceptable.

As set forth above, the band device 10 is particularly adapted to be laparoscopically implanted. In this regard, although the device disclosed in Application Serial No. 08/019,302 filed on February 18, 1993, can also be laparoscopically implanted and, in this regard, is not limited to any particular method of implantation, the differences between the device of the present application and that of the earlier application, and more specifically, the increased flexibility and the thinner, more streamlined shape of the gastric band device of the invention facilitate laparoscopic implantation thereof. In general, the laparoscopic procedure used to implant the device is that disclosed in Serial No. 07/179,123, filed on June 20, 1991. In fact, the only differences in the procedure flow from the differences in the two band devices. In this regard, the band 12 of the invention would be placed around the stomach in the same general way after using an endoscope introduced into a cannula to dissect tissue surrounding the stomach so as to provide a path for the gastric band 12. However, in contrast to the earlier device, the remotely controlled movement of the control member 10 would be used to provide locking of the locking mechanism (comprising locking element or projection 20 and slot or recess 22), thus fixing band 12 in place in an encircling relation around the stomach. The injection unit 28, located at the end of the band 12, would be implanted in the abdominal wall muscles, as in the prior application, and the overall laparoscopic procedure would likewise be similar.

Considering methods of removal of the band 12 after implantation, as indicated above, the living body in which the device of the invention is inserted creates a thin capsule around the implanted silicone band 12 that is not adhered to the band 12. By exposing the site of the injection unit implantation, i.e., the site at which unit 28 is disposed within the abdominal wall muscles, the proximal end of the band 12 is exposed as well. Through displacement of the threaded bolt 30 by remotely controlled movement of control member 18, the band locking mechanism (comprising locking element 20 and slot or recess 22) can be released.

In accordance with one method of removing the band, the control member 18 and the locking bolt 30, which are stiff and difficult to cut relative to elongate band portion 12c, are removed sufficiently from the band 12, as shown in Figure 5(a), so that the elongate portion 12c, i.e., the portion not containing the recess 22 or the expandable section 24, may be easily cut close to recess 22, without cutting the control member 18. This allows the relatively thin band portion 12c to be removed with the injection unit 28 through the site of the injection unit implantation, preferably in the abdominal muscles near the skin. The remainder of the SASGB, i.e., portion 12b containing the recess 22 and portion 12a containing the expandable section 24, can then be removed laparoscopically at the site of the these portions of the band 12, i.e. at the stoma opening.

Alternatively, referring to Figure 5(b), the SASGB of the present invention can also be removed by completely removing the control member 18 from within the band 12 and by removing the injection port 28 through a small superficial incision. With these components removed, the entire SASGB, including the relatively thin elongate portion 12c, is then removed laparoscopically at the site of the stoma. One advantage of removing the band or a part thereof at the stoma is that the surgeon may wish to gain access to this area in any event in order to remove suturing at the stoma.

As should be evident from the foregoing, the band device 10 of the present invention is more effective than the prior art in uniformly controlling the size of stoma opening and, among other advantages, is less invasive to the stomach wall because of the use of the inflatable part 24 which covers the entire portion of band 12a that encircles the stomach. The use of the thinner elongate portion 12C also helps to relieve the discomfort felt by some patients when the elongate portion 12C is of a greater thickness.

Although the present invention has been described relative to specific exemplary embodiments thereof, it will be understood by those skilled in the art that variations and modifications can be effected in these exemplary embodiments without departing from the scope of the invention as defined by the claims.

## Claims

1. A gastric banding device (10) for encircling a stomach (S) to form a restricted stoma opening in the stomach (S) so as to restrict food intake to a lower digestive portion thereof, said device (10) comprising:
a band member (12) including a band portion (12a) for encircling the stomach (S) so as to form the restricted stoma opening, the band portion (12a) having a free end, wherein the band member (12) further includes an expandable section (24) secured to the band portion (12a) and comprising an elongate inflatable member (24) extending completely along the band portion (12a) such that the expandable section (24) fully encircles the stomach (S) when the device (10) is in place forming the restricted stoma opening; and being
characterised by
a first interlocking securing means (20) disposed at a first predetermined fixed position on the band portion (12a) close to said free end, and a second interlocking securing means (22) disposed at a second predetermined fixed position located in an intermediate portion (12b) of the band member (12) for interlocking with the first interlocking securing means (20) to provide securing of the said free end of the band portion (12a) to the intermediate portion (12b) thereof at said second position so that the band portion (12a) forms a circle of predetermined fixed diameter which is predetermined by the locations of the first and second fixed positions on the band member (12).

2. A gastric banding device (10) as claimed in claim 1, wherein the expandable section (24) comprises reinforced walls.

3. A gastric banding device (10) as claimed in claim 1 or claim 2, further comprising means (28) for controlling expansion and contraction of the expandable section (24) so as to control the size of the restricted stoma opening, the means (28) comprising a remotely disposed fluid injection unit (28) in fluid connection with the expandable section (24) through the band member (12).

4. A gastric banding device (10) as claimed in any preceding claim, wherein the first interlocking securing means (20) comprises a securing member (20) located close to the free end of the band portion (12a) and protruding outwardly therefrom, and the second interlocking securing means (22) comprises a recess (22) provided in the intermediate portion (12b) of the band member (12) for receiving the securing member (20) therein, and wherein the gastric banding device (10) further comprises retaining means (30) for retaining the securing member (20) in the recess (22).

5. A gastric banding device (10) as claimed in claim 4, further comprising barrier means (40) on the band member (12) for preventing tissue from growing around the securing member (20).

6. A gastric banding device (10) as claimed in claim 5, wherein the barrier means (40) comprises a soft sponge-like silicone barrier element (40).

7. A gastric banding device (10) as claimed in any one of claims 4 to 6, further comprising a thin loop of silicone (38) disposed around the securing member (20) for ensuring a secure fit between the securing member (20) and the recess (22).

8. A gastric banding device (10) as claimed in any one of claims 4 to 7, further comprising an elongate control element (18) located within the band member (12) and extending longitudinally thereof, the control element (18) including a distal end
and a proximal end which extends beyond the band member (12) for allowing remotely controlled release of the securing member (20) from the recess (22) by movement of the retaining means (30).

9. A gastric banding device (10) as claimed in any one of claims 4 to 8, wherein the retaining means (30) comprises a moveable bolt (30).

10. A gastric banding device (10) as claimed in claim 9, wherein the bolt (30) has a free end, the device (10) further including a guiding plate (34) located within the band member (12) on one side of the recess (22) for guiding the movement of the bolt (30), and a holding plate (32) located within the band member (12) on the other side of the recess (22) for retaining the free end of the bolt (30).

## Patentansprüche

1. Magenband-Vorrichtung (10) zum Umschließen eines Magens (S), um eine eingeschränkte Stomaöffnung im Magen (S) zu bilden, so daß die Nahrungsaufnahme auf einen unteren Verdauungsabschnitt davon beschränkt ist, wobei die Vorrichtung (10) umfaßt:
ein Bandelement (12), das einen Bandabschnitt (12a) zum Umschließen des Magens (S) umfaßt, um die eingeschränkte Stomaöffnung zu bilden, wobei der Bandabschnitt (12a) ein freies Ende aufweist, worin das Bandelement (12) weiters einen ausdehnbaren Abschnitt (24) umfaßt, der am Bandabschnitt (12a) befestigt ist und ein längliches aufblasbares Element (24) umfaßt, das sich vollständig den Bandabschnitt (12a) entlang erstreckt, so daß der ausdehnbare Abschnitt (24) den Magen (S) vollständig umschließt, wenn die Vorrichtung (10) sich in Position befindet, um die eingeschränkte Stomaöffnung zu bilden; und gekennzeichnet durch
ein erstes verriegelndes Befestigungsmittel (20), das auf dem Bandabschnitt (12a) nahe dem freien Ende an einer ersten vorbestimmten fixen Position angeordnet ist, sowie ein zweites verriegelndes Befestigungsmittel (22), das in einer zweiten vorbestimmten fixen Position angeordnet ist, die sich in einem Mittelabschnitt (12b) des Bandelements (12) befindet, um mit dem ersten verriegelnden Befestigungsmittel (20) ineinanderzugreifen, so daß für Befestigung des freien Endes des Bandabschnitts (12a) an seinem Mittelabschnitt (12b) in einer zweiten Position gesorgt wird, so daß der Bandabschnitt (12a) einen Kreis mit einem vorbestimmten fixen Durchmesser bildet, der durch die Positionen der ersten und der zweiten fixen Position auf dem Bandelement (12) vorbestimmt ist.

2. Magenband-Vorrichtung (10) nach Anspruch 1, worin der ausdehnbare Abschnitt (24) verstärkte Wände umfaßt.

3. Magenband-Vorrichtung (10) nach Anspruch 1 oder 2, die weiters Mittel (28) zum Regulieren der Ausdehnung und Kontraktion des ausdehnbaren Abschnitts (24) umfaßt, um die Größe der eingeschränkten Stomaöffnung zu regulieren, wobei das Mittel (28) eine entfernt angeordnete Fluideinspritzeinheit (28) umfaßt, die sich durch das Bandelement (12) in Fluidverbindung mit dem ausdehnbaren Abschnitt (24) befindet.

4. Magenband-Vorrichtung (10) nach einem der vorangegangenen Ansprüche, worin das erste verriegelnde Befestigungsmittel (20) ein Befestigungselement (20) umfaßt, daß nahe dem freien Ende des Bandabschnitts (12a) angeordnet ist und von diesem nach außen ragt, und das zweite verriegelnde Befestigungsmittel (22) eine Ausnehmung (22) umfaßt, die im Mittelabschnitt (12b) des Bandelements (12) vorgesehen ist, um das Befestigungselement (20) darin aufzunehmen, und worin die Magenband-Vorrichtung (10) weiters Rückhaltemittel (30) umfaßt, um das Befestigungsmittel (20) in der Ausnehmung (22) zu halten.

5. Magenband-Vorrichtung (10) nach Anspruch 4, die weiters Barrieremittel (40) auf dem Bandelement (12) umfaßt, um zu verhindern, daß das Befestigungselement (20) von Gewebe umwachsen wird.

6. Magenband-Vorrichtung (10) nach Anspruch 5, worin das Barrieremittel (40) ein weiches schwammartiges Silikon-Barriereelement (40) umfaßt.

7. Magenband-Vorrichtung (10) nach einem der Ansprüche 4 bis 6, die weiters eine dünne Silikonschlaufe (38) umfaßt, die um das Befestigungselement (20) herum angeordnet ist, um eine sichere Passung zwischen dem Befestigungselement (20) und der Ausnehmung (22) zu gewährleisten.

8. Magenband-Vorrichtung (10) nach einem der Ansprüche 4 bis 7, die weiters ein längliches Regulierungselement (18) umfaßt, das innerhalb des Bandelements (12) angeordnet ist und sich in Längsrichtung davon erstreckt, wobei das Regulierungselement (18) ein distales Ende
sowie ein proximales Ende umfaßt, das sich über das Bandelement (12) hinaus erstreckt, um die ferngesteuerte Freigabe des Befestigungselements (20) aus der Ausnehmung (22) durch Bewegen des Rückhaltemittel (30) zuzulassen.

9. Magenband-Vorrichtung (10) nach einem der Ansprüche 4 bis 8, worin das Rückhaltemittel (30) eine bewegliche Schraube (30) umfaßt.

10. Magenband-Vorrichtung (10) nach Anspruch 9, worin die Schraube (30) ein freies Ende aufweist, wobei die Vorrichtung (10) weiters eine Führungsplatte (34) umfaßt, die sich innerhalb des Bandelements (12) an einer Seite der Ausnehmung (22) befindet, um die Bewegung der Schraube (30) zu führen, sowie eine Halteplatte (32), die sich innerhalb des Bandelements (12) an der anderen Seite der Ausnehmung (22) befindet, um das freie Ende der Schraube (30) zurückzuhalten.

## Revendications

1. Dispositif formant bande gastrique (10) pour encercler un estomac (S) pour former une ouverture restreinte de stoma dans l'estomac (S) de façon à restreindre l'admission de la nourriture vers une partie digestive inférieure de celui-ci, ledit dispositif (10) comprenant :
un élément de bande (12) incluant une portion de bande (12a) pour encercler l'estomac (S) de façon à former l'ouverture de stoma restreinte, la portion de bande (12a) ayant une extrémité libre, où l'élément de bande (12) comporte en outre une section d'expansion (24) fixée à la portion de bande (12a) et comprenant un élément oblong gonflable (24) s'étendant complètement le long de la portion de bande (12a) de telle sorte que la section d'expansion (24) encercle complètement l'estomac (S) lorsque le dispositif (10) est en place formant l'ouverture de stoma restreinte ; et étant caractérisé par
un premier moyen de fixation d'interverrouillage (1) disposé à une première position prédéterminée fixe sur la portion de bande (12a) à proximité de ladite extrémité libre, et un deuxième moyen de fixation d'interverrouillage (22) disposé à une deuxième position prédéterminée fixe située dans une portion intermédiaire (12b) de l'élément de bande (12) pour l'interverrouillage avec le premier moyen de fixation d'interverrouillage (20) pour permettre la fixation de ladite extrémité libre de la portion de bande (12a) à la portion intermédiaire (12b) de celle-ci à ladite deuxième position de telle sorte que la portion de bande (12a) forme un cercle d'un diamètre prédéterminé fixe qui est prédéterminé par les emplacements des première et deuxième positions fixes sur l'élément de bande (12).

2. Dispositif formant bande gastrique (10) tel que revendiqué dans la revendication 1, où la section d'expansion (24) comprend des parois de renforcement.

3. Dispositif formant bande gastrique (10) tel que revendiqué dans la revendication 1 ou la revendication 2, comprenant en outre un moyen (28) pour contrôler l'expansion et la contraction de la section d'expansion (24) de façon à contrôler la taille de l'ouverture de stoma restreinte, le moyen (28) comprenant une unité d'injection de fluide (28) disposée à distance en connexion de fluide avec la section d'expansion (24) par l'élément de bande (12).

4. Dispositif formant bande gastrique (10) selon l'une des revendications précédentes, où le premier moyen de fixation d'interverrouillage (20) comprend un élément de fixation (20) situé à proximité de l'extrémité libre de la portion de bande (12a) et faisant saillie vers l'extérieur à partir de celle-ci, et le deuxième moyen de fixation d'interverrouillage (22) comprend un évidement (22) ménagé dans la portion intermédiaire (12b) de l'élément de bande (12) pour recevoir l'élément de fixation (20) dans celui-ci, et où le dispositif formant bande gastrique (10) comporte en outre un moyen de retenue (30) pour retenir l'élément de fixation (20) dans l'évidement (22).

5. Dispositif formant bande gastrique (10) selon la revendication 4, comprenant en outre un moyen de barrière (40) sur l'élément de bande (12) pour empêcher une croissance du tissu autour de l'élément de fixation (20).

6. Dispositif formant bande gastrique (10) selon la revendication 5, où le moyen de barrière (40) comprend un élément de barrière souple, en forme d'éponge en silicone (40).

7. Dispositif formant bande gastrique (10) selon l'une des revendications 4 à 6, comprenant en outre une boucle mince en silicone (38) disposée autour de l'élément de fixation (20) pour assurer une adaptation sûre entre l'élément de fixation (20) et l'évidement (22).

8. Dispositif formant bande gastrique (10) selon l'une des revendications 4 à 7, comprenant en outre un élément de contrôle oblong (18) situé dans l'élément de bande (12) et s'étendant longitudinalement à partir de celui-ci, l'élément de contrôle (18) incluant une extrémité distale et une extrémité proximale qui s'étend au-delà de l'élément de bande (12) pour permettre un relâchement commandé à distance de l'élément de fixation (20) de l'évidement (22) par un déplacement du moyen de retenue (30).

9. Dispositif formant bande gastrique (10) selon l'une des revendications 4 à 8, où le moyen de retenue (30) comprend un boulon déplaçable (30).

10. Dispositif formant bande gastrique (10) selon la revendication 9, où le boulon (30) a une extrémité libre, le dispositif (10) incluant en outre une plaque de guidage (34) située dans l'élément de bande (12) sur un côté de l'évidement (22) pour guider le déplacement du boulon (30), et une plaque de retenue (32) située dans l'élément de bande (12) de l'autre côté de l'évidement (22) pour retenir l'extrémité libre du boulon (30).
